# EUROPEAN PATENT APPLICATION

(11) **EP 3 971 295 A1**
(43) Date of publication of application: **23.03.2022**
(21) Application number: 20196437.6
(22) Date of filing: 16.09.2020
(51) Int. Cl.: C12N 15/82

(54) **METHODS FOR THE PRODUCTION OF GENOME EDITED PLANTS**

(71) Applicant: FRAUNHOFER-GESELLSCHAFT zur Förderung der angewandten Forschung e.V., 80686 München (DE)
(72) Inventor: AUGUSTINE, Sruthy Maria, 52070 Aachen (DE); VADAKAN CHERIAN, Anoop, 35392 Giessen (DE); SEILING, Kerstin, 59510 Lippetal (DE); RAVEN, Nicole, 35392 Giessen (DE); DI FIORE, Stefano, 41460 Neuss (DE); SCHILLBERG, Stefan, 52074 Aachen (DE); COMMANDEUR, Ulrich, 52074 Aachen (DE)
(74) Representative: Roth, Andy Stefan

(57) **Abstract**

The technology provided herein relates to the generation of DNA free genome edited plants and the delivery of pre-assembled ribonucleoprotein complexes (RNP) - such as the Cas9/gRNA RNP complex - for highly efficient genome editing of cells, in particular of intact plant cells and tissues, selection of the transfected cells and regeneration of whole plants thereof.

## Description

### FIELD OF THE DISCLOSURE

The technology provided herein relates to methods for highly efficient, mild and fast generation of genome edited intact plant cells and regeneration of cell lines, whole tissues or organisms thereof.

### BACKGROUND

The variety of traits found in nature reflects the presence of one or multiple mutations in certain genes, which often arise as mistakes in the repair mechanism initiated after DNA damage. To expand the repertoire of traits readily available for breeding, methods for artificially changing genomes by means of mutations induced by chemicals and radiation have been developed. More recently, strategies such as tilling (Targeting Induced Local Lesions in Genomes) have been developed as reverse genetics tools, which also entail the use of a chemical or radiation mutagens, causing extensive DNA damage and therefore, mutations. These classical approaches considerably expanded the range of practically useful traits available for breeding and accelerated the establishment and release of improved plant varieties. However, as the mutations are induced randomly, extensive screening efforts to identify the mutation of interest are still needed. In addition, as many mutations are induced simultaneously, after successful identification of a line displaying the desired trait, several rounds of backcrossing are needed to eliminate most of the background mutations. With the discovery of site-directed nucleases (SDNs) and the advent of genome editing it is now possible to induce a break, and therefore a mutation, at specific pre-determined locations in the genome. These locations in the genome are defined by their sequence and the sequence specificity of the DNA-modifying enzymes. In fact, when a double strand break occurs in a higher eukaryotic cell, it is often repaired via the Non-Homologous End Joining (NHEJ) pathway resulting in small insertions or deletions (indels) which mutate the targeted gene. If a short piece of double or single stranded DNA homologous to the targeted region but carrying a single or few base mutations is provided along with the nuclease, the break can be repaired by the Homology-Directed Repair (HDR) pathway, allowing to precisely edit a few nucleotides in the target gene.

The latest ground-breaking genome editing technology is based on RNA-guided engineered nucleases, which have rapidly set themselves as a very efficient and versatile tool. Currently, the most widely used system is the type II clustered regularly interspaced short palindromic repeat (CRISPR)/Cas9 (CRISPR-associated) system from *Streptococcus pyogenes.* The natural system comprises small interfering CRISPR RNAs (crRNAs) approximately 40 nucleotides in length, which combine with the transactivating CRISPR RNA (tracrRNA) to activate and guide the Cas9 nuclease. This cleaves homologous double-stranded DNA sequences known as protospacers in the invading DNA. A prerequisite for cleavage is the presence of a conserved protospacer-adjacent motif (PAM) downstream of the target DNA, which usually has the sequence 5'-NGG-3'. The target DNA sequence can be reprogrammed simply by changing 20 nucleotides in the crRNA and the targeting specificity of the crRNA can be combined with the structural properties of the tracrRNA in a chimeric single guide RNA (gRNA) (Jinek et al., 2012).

Since their first discovery, SDNs including the CRISPR/Cas9 components (Cas9 and single guide RNA) have been introduced into plants as DNA (plasmid, dsDNA fragment or single-stranded T-DNA), generating *de facto* transgenic plants or transgenic intermediates (when the transgene is segregated out or eliminated in a subsequent step). However, production of transgenic plants is a very controversial topic, with public opinion often being against the creation of such varieties, particularly for crop plants that are cultivated over large geographical areas and used as food for human consumption. Even in the case where the transgene is segregated out via crossing or is delivered as a plasmid or viral replicon and expressed only transiently in the plant cell, it is possible that the DNA or small fragments thereof are inserted at random position in the genome. These can be detected with certainty only by whole genome sequencing to be able to ultimately determine if the resulting plants falls into the transgenic category (Kim and Kim 2016). However, whole genome sequencing is a rather expensive and time-consuming procedure and, most importantly, it cannot be performed for species for which a reference genome is not available in the first place. Moreover, the question whether or not a genome modified plant has to be considered as transgenic can depend on whether or not recombinant DNA has been used in the process rather than whether this recombinant DNA is comprised in the final organism.

Direct delivery of RNP complexes for genome editing is highly desirable because it does not involve exogenous DNA, precluding the possibility of the resulting plant to be considered transgenic. The problem of delivering RNP complexes into plant cells is that these cells are surrounded by a cell wall, which hinders the entry of large molecules inside the cell. Gene editing by direct delivery of the CRISPR RNP complex into protoplasts via PEG transfection and regeneration of plants thereof has been described (Woo et al., 2015). However, obtaining protoplasts from plant tissues and then regenerating whole plants from single protoplasts is a very delicate procedure that has to be optimized for each plant species (in some cases even cultivar), and it is well established only for a handful of species. Notably, many important crops cannot be regenerated from protoplasts efficiently.

In contrast, regeneration of whole plants from several tissues is a more straightforward procedure for many more species, including some of major economic relevance. Direct delivery of RNP complexes into maize embryo cells and bread wheat embryo cells by particle bombardment and regeneration of plants thereof has also been reported (Svitashev et al., 2016; Liang et al., 2017). Yet, in many cases only particular cultivars are performing well in tissue culture, thus making subsequent breeding into agronomical important cultivars necessary. Tissue culture and particularly the generation of dedifferentiated cells often induces somaclonal variations and epigenetic effects, hence methods reducing or avoiding these steps are also highly desirable.

Biolistic delivery methods furthermore are technically challenging, require sophisticated and expensive equipment, and largely depend on sterile tissue culturing steps for generating genetically modified and genome-edited organisms. The dependence on sterile tissue culture techniques, which typically involve cell dedifferentiation by hormones, not only limits the application of particle bombardment, but also increases the chances to acquire undesired epigenetic effects and somaclonal variations in the resulting regenerated organisms. This then can make additional breeding steps necessary, which are time consuming and costly. Therefore, novel delivery methods that can directly be used on differentiated cells, preferably on the intact organism grown under non-sterile conditions, or methods that are less dependent on tissue culture steps, the use of hormones, or avoid these steps altogether are highly desirable.

Another problem associated with many delivery methods, and particularly with particle bombardment, is that delivery of the cargo is limited to a few cells only, i.e. that the overall efficiency is low. The few cells that have received the cargo are present in between a large number of cells that have not received the cargo. When taking samples for analysis, the percentage of cells that are affected by the treatment is usually not known, which can significantly obscure and confound the analysis. For the same reasons, the regeneration of cell lines, whole tissue or organisms from the treated cells is laborious and time consuming. Many experiments are performed, and the treated cells are incubated over prolonged periods of time to ensure that at least a few events, i.e. genome-edited cell lines, whole tissues or organisms, are obtained. Thus, there is a need in the field for novel methods that offer simpler and more effective ways of delivering genome-modifying formulations, e.g. comprising ribonucleoprotein (RNP)-complexes, into intact cells and in particular into differentiated plant cells with a cell wall for a wide variety of plant species. In addition, simpler and more effective ways to identify the treated cells are needed to reduce the workload and increase the overall efficiency of obtaining genome-edited cell lines, whole tissues or organisms from the treated cells.

Methods that have a higher overall regeneration efficiency for generating genome-edited cell lines, whole tissues or organisms without introducing exogenous DNA are also highly desirable and a prerequisite for modifying two or more target regions at the same time or performing more advanced modifications of the genome.

### SUMMARY OF THE DISCLOSURE

The present disclosure pertains to methods for highly efficient, mild and fast generation of genome edited intact and differentiated plant cells and regeneration to cell lines, whole tissues or organisms thereof

Further, the present disclosure pertains to methods for the delivery of genome-modifying formulations, such as pre-assembled ribonucleoprotein complexes (RNP) - like the Cas9/gRNA RNP complex - for highly efficient genome editing to intact differentiated cells and regeneration to cell lines, whole tissues or organisms thereof. Furthermore, the present disclosure pertains to methods to produce, select and regenerate genome edited plants i.e. without inserting exogenous genetic material into the genome of the plant. In particular, the methods according to the present disclosure may solve the following technical problems related to DNA-free genome editing in plants:
- facilitates the introduction of RNP in intact plant cells and shows high efficiency compared to existing methods
- accelerate screening of mutants without requiring selection markers
- it is gentle and therefore harnesses a high efficiency of plant regeneration from plant leaf explants compared to existing methods within a short period of time (less than 8 months)

In particular, with the methods according to the present disclosure pre-assembled RNP complexes are delivered through the cell wall into the cell by a laser-assisted transfection method, in particular wherein the ribonucleic acid in the RNP-complex is fluorescent-labelled.

In a first aspect, embodiments of the disclosure provide novel methods for altering the genome of an intact plant cell(s) without inserting exogenous genetic material into the genome comprising:
i) providing an intact plant cell that comprises an endogenous gene to be modified;
(ii) providing a genome-modifying formulation comprising a pre-assembled ribonucleoprotein (RNP)-complex comprising a nucleic acid modifying protein and ribonucleic acid, wherein the ribonucleic acid is labeled with a visual marker;
(iii) delivering said RNP-complex into the plant cell with a laser-assisted transfection method, wherein the cell is surrounded by a liquid and the laser focal point is in the liquid adjacent to the cell wall and not directly in contact with the cell wall;
(iv) inducing one or more single or double stranded DNA breaks in the cell genome to produce a cell having a detectable targeted genomic modification without the presence of any exogenous genetic material in the cell genome.

In a second aspect, embodiments of this disclosure relate to methods for delivering a ribonucleoprotein (RNP)-complex into cells comprising the steps of:
(i) providing an intact plant cell;
(ii) providing a genome-modifying formulation comprising a pre-assembled ribonucleoprotein (RNP)-complex comprising a nucleic acid modifying protein and a ribonucleic acid, wherein the ribonucleic acid is labeled with a visual marker;
(iii) delivering said RNP-complex into the plant cell with a laser-assisted transfection method, wherein the cell is surrounded by a liquid and the laser focal point is in the liquid adjacent to the cell wall and not directly in contact with the cell wall;
(vi) utilizing the labeled ribonucleic acid to identify the cell, the cells or the areas within a tissue, organ or whole organism to which the genome-modifying formulation has been delivered, followed by selecting those cell(s) or areas for deriving a genome-modified cell line, whole tissue or organism thereof.

In a third aspect, embodiments of this disclosure relate to methods to produce genome edited plants without inserting exogenous genetic material into the genome of the plant comprising the steps of:
(i) providing a plant explant isolated by any organ and tissue of a plant and comprising intact plant cells comprising an endogenous gene to be modified;
(ii) providing a genome-modifying formulation comprising a pre-assembled ribonucleoprotein (RNP)-complex comprising a nucleic acid-modifying protein and ribonucleic acid, wherein the ribonucleic acid is labeled with a visual marker;
(iii) layering the plant explant onto the RNP-complex formulation in a transparent support;
(iv)delivering said RNP-complex into a plant cell of the plant explant with laser-assisted transfection, wherein the cell is surrounded by a liquid and the laser focal point is in the liquid adjacent to the cell wall and not directly in contact with the cell wall;
(v) inducing one or more single or double stranded DNA breaks in the cell genome to produce a cell having a detectable targeted genomic modification without the presence of any exogenous genetic material in the cell genome.
(vi) identifying the cells transfected with the RNP-complex by microscopy, in particular by fluorescence microscopy;
(vii) selecting the cells transfected with the RNP-complex from the plant explant
(viii) regenerating the cells to intact plantlets by growing in and/or on a growth media.

In particular, the methods according to the present disclosure enables the use of "cells obtained from non-sterile environment and/or provided under non-sterile environment". In the prior art it is mentioned that cells grown in sterile tissue culture differ from cells obtained from non-sterile environments in many aspects. Cells cultivated in sterile tissue culture are dividing, and often they are (partly) dedifferentiated, treated with growth hormones, and generally have different intracellular states (metabolism, gene expression, ...). Phenomena such as soma-clonal variation, epigenetic changes, activation of transposons, promotor methylation, gene activation / deactivation, are all well known in the art and are of concern in breeding programs, genetic engineering and genome editing. Many species are difficult to maintain in tissue culture, and often this is restricted to particular cultivars only. For many reasons it is therefore highly desirable to reduce the amount of tissue culture as much as possible or to avoid it altogether.

The method of the present disclosure may in particular comprise a sequential application of non-invasive techniques including
1. laser-assisted transfection of e.g. fluorescent-labelled RNPs in intact plant cells,
2. optically aided selection and excision of transfected plant cells within plant tissue explants,
3. regeneration of plantlets and
4. genetic profiling and screening of mutants by e.g. High Resolution Melt Analysis (HRMA).

It is an advantage of the methods of the present disclosure that RNPs can be efficient and gentle delivered into cells for an effective regeneration of DNA-free genome-edited plants. The method of the present disclosure are largely advantageous over contemporary methods, such as particle bombardment, electroporation, PEG-mediated transfection and microinjection because it overcomes all the collective limitations of these alternative methods.

Unlike PEG-mediated transfection, the present methods allows the direct introduction of RNPs into intact plant cells rather than protoplasts, thus reducing the time required for regeneration and making the method accessible in species that cannot yet regenerate from protoplasts. Unlike other physical delivery techniques, the present methods are gentle because the fine-tuned laser irradiation does not affect cell viability and preserves a larger population of intact transfected cells, making it easier to isolate transfected cells and tissue segments for regeneration.

One of the most important aspects according to the present methods is the flexibility because the principles of the methods are universally applicable to any explant of any plant species that can be regenerated in tissue culture. The methods are particularly advantageous for the development of DNA-free genome-edited plants/crops of different species in a very short period, particularly for those species that cannot be regenerated via protoplasts or that are particularly difficult to transfect with current methods.

Efficient delivery of RNPs is required for effective DNA-free genome editing, and the methods of the present disclosure are mainly based on laser-assisted transfection method, wherein the cell is surrounded by a liquid and the laser focal point is in the liquid adjacent to the cell wall and not directly in contact with the cell wall, in particular in combination with a labeled RNP complex. The method of the present disclosure is e.g. advantageous over contemporary methods such as particle bombardment, electroporation, PEG-mediated transfection and microinjection because it overcomes all the collective limitations of these alternative methods. Unlike PEG-mediated transfection for example, the present method allow the direct introduction of RNPs into intact plant cells rather than protoplasts, thus reducing the time required for regeneration and making the method accessible in species that cannot yet regenerate from protoplasts or from tissues which underwent the harsh conditions of particle bombardment. Unlike other physical delivery techniques, the method of the present disclosure is gentle and therefore preserves a larger population of intact transfected cells, making it easier to isolate transfected cells and tissue segments for regeneration.

One of the most important aspects of the present disclosure is the development of exogenous DNA-free genome-edited plants/crops of different species in a very short period of time, potentially any plant species that can be regenerated in tissue culture, because the principle of the method is universally applicable to any plant explant.

Before the disclosure is described in detail, it is to be understood that this disclosure is not limited to the particular component parts of the process steps of the method described. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. It is moreover to be understood that, in case parameter ranges are given which are delimited by numeric values, the ranges are deemed to include these limitation values.

### BRIEF DESCRIPTION OF THE DRAWINGS

Figure 1: A) Schematic representation of the laser setup. B) Representative leaf sample used for DsRed/RNP introduction. Scale bar = 1 cm.
Figure 2: A) Tobacco leaves before the laser-assisted treatment. The white arrow points to the region chosen for laser targeting. Scale bar = 50 µm. A') The same region after the laser-assisted uptake of 20 µL DsRed solution (0.25 µg/µL). DsRed fluorescence was detected by confocal microscopy. A") 3D reconstruction of DsRed-positive cells after laser treatment. Scale bar = 50 µm.
Figure 3: *Zea mays* leaves before and after the laser-assisted transfection. White arrows point to the region chosen for laser targeting. Scale bar = 50 µm.
Figure 4: A) Detection of ATTO-550-labeled fluorescent RNPs in cells after laser-assisted transfection. Wild-type plants do not show any fluorescence. Scale bar = 50 µm. B) Representative sampling of RNP-containing regions of different sizes (^{∼}0.1-0.3 cm in radius) depending on the intensity of the RNP signal. Scale bar = 1 cm.
Figure 5: Albino *pds* -/- homozygous mutant lines T10, 126, F26 and L35 generated with the method of the present invention and compared to a wild-type plant.
Figure 6: A) Representative image showing the high-resolution melt analysis of a pds mutant (line T10) generated and selected with the method of the present invention. B) Sequence alignments of genome-edited mutants. C) Representative chromatogram of wild-type and pds mutant plant (line T10).
Figure 7: A) Representative image showing the high-resolution melt analysis of an *adf* mutant (line A59) generated and selected with the method of the present invention. B) Sequence alignment of genome-edited mutants. C) Exemplary chromatogram analysis of wild-type (wt) and mutant plant (line A59).
Figure 8: A) Representative image of wt and *adf* mutant plants of the present invention with corresponding phalloidin stained cells. Scale bar = 50 µm. B) Image analysis of average actin intensity normalized by the area of fluorescence in wt and *adf* mutant (line A59) cells using ImageJ.

### DETAILED DESCRIPTION OF THE DISCLOSURE

The present disclosure pertains to the generation of DNA free genome edited plants and the delivery of pre-assembled ribonucleoprotein complexes (RNP) - such as the Cas9/gRNA RNP complex - for highly efficient genome editing of cells, in particular of intact plant cells and tissues, selection of the transfected cells and regeneration of whole plants thereof. The RNP complex is allowed to form *in vitro* and then is delivered through the plant cell wall by a laser-assisted transfection method, wherein the cell is surrounded by a liquid and the laser focal point is in the liquid adjacent to the cell wall and not directly in contact with the cell wall and/or the plant cell in general. In particular, the ribonucleic acid in the pre-assembled ribonucleoprotein (RNP)-complex is labeled with a visual marker, in particular the ribonucleic acid is fluorescent-labelled.

The present disclosure pertains to the production of genome edited plants without inserting exogenous genetic material into the genome of the plant by the delivery of pre-assembled ribonucleoprotein complexes (RNP) - such as the Cas9/gRNA RNP complex - for highly efficient genome editing of cells, in particular of intact plant cells and regeneration of whole plants thereof. The RNP complex is allowed to form *in vitro* and then is delivered through the plant cell wall by a laser-assisted transfection method, wherein the cell is surrounded by a liquid and the laser focal point is in the liquid adjacent to the cell wall and not directly in contact with the cell wall and/or the plant cell. In particular, the ribonucleic acid in the pre-assembled ribonucleoprotein (RNP)-complex is labeled with a visual marker; in particular the ribonucleic acid is fluorescent-labelled.

For example, the "CRISPR/Cas" system refers to a widespread class of bacterial systems for defense against foreign nucleic acid. CRISPR/Cas systems are found in a wide range of eubacterial and archaeal organisms. CRISPR/Cas systems include type I, II, and III sub-types. Wild-type type II CRISPR/Cas systems utilize an RNA-mediated nuclease, Cas9 in complex with guide and activating RNA to recognize and cleave foreign nucleic acid. Guide RNAs having the activity of both a guide RNA and an activating RNA are also known in the art. In some cases, such dual activity guide RNAs are referred to as a small guide RNA (sgRNA).

By delivering the RNP as a pre-formed complex, the editing efficiency is so high that whole plants carrying mutations at the desired target site can be regenerated and recovered without applying any selection. In contrast to the described procedure of delivering the RNP complex by protoplast transfection, with the present methods eukaryotic cells like plant cells (i.e. suspension cells, calli, leaves, stems, cotyledons, pollen, etc.) can be readily edited without the need of going through the protoplast stage. As not all plant species are amenable to protoplasting, and regeneration of whole plants from protoplasts is technically difficult, this is a major improvement that greatly simplifies and most importantly extends the use of DNA-free genome editing to basically any plant species that can regenerate from tissue culture.

DNA-free genome editing is highly desirable as it may be exempt from GMO regulations and the ability to use proteins or pre-assembled ribonucleoprotein complexes (RNP) facilitates an entire range of application possibilities.

In one aspect, the present disclosure provides methods for altering the genome of an intact differentiated cell (e.g. altering or modifying expression of one or more gene products).

Several methods have been previously described and traditionally used to deliver nucleic acids in association with a selection agent to identify and/or recover the events of interest, or have been used to introduce self-replicating viruses or viral replicons that only require a minimal starting inoculum to infect a whole plant. Indeed, to achieve genome-editing in plants biolistic methods based on the bombardment of RNPs or delivery of a fluorescent protein into intact plant tissues coated onto e.g. gold nanoparticles have been previously described (Bortesi et al., 2017, Martin-Ortigosa et al., 2014). However, only a few spots of fluorescence where visible within the bombarded tissue area and the accelerated gold nanoparticles caused damage to the plant cells thereby significantly reducing the efficiency of regeneration of whole plants from the transfected cells. The delivery of the RNPs by the laser-assisted transfection of the present invention is gentle and this improves the regeneration rate of genome-edited plants as compared to harsher methods such as particle bombardment.

Alternatively, methods for RNP delivery have been described for protoplasts by the use of chemical transfection agents, such as polyethylene glycol (PEG). Compared to delivery of RNPs to protoplasts, the present invention greatly simplifies the procedure to obtain genome edited plants because all the steps necessary to obtain and regenerate the protoplasts are not necessary. Thereby, the present invention extends the procedure of RNP complex delivery to many more plant species which are not amenable to regenerate from protoplasts, i.e. to basically any plant species that can regenerate from tissue culture. The present invention facilitates genome editing to more plant species, in a faster and more efficient manner and represents a significant competitive advantage for generating and breeding the next generations of crops and designer-plants for various purposes.

In an advantageous embodiment, the present disclosure pertains to a method for altering the genome of an intact plant cell without inserting exogenous genetic material into the genome comprising:
(i) providing an intact plant cell that comprises an endogenous gene to be modified;
(ii) providing a genome-modifying formulation comprising a pre-assembled ribonucleoprotein (RNP)-complex comprising a nucleic acid-modifying protein and ribonucleic acid, wherein the ribonucleic acid is labeled with a visual marker;
(iii) delivering said RNP-complex into the plant cell with a laser-assisted transfection method, wherein the cell is surrounded by a liquid and the laser focal point is in the liquid adjacent to the cell wall and not directly in contact with the cell wall;
(iv) inducing one or more single or double stranded DNA breaks in the cell genome to produce a cell having a detectable targeted genomic modification without the presence of any exogenous genetic material in the cell genome.

An important aspect of the present disclosure is that the RNP complex is first allowed to assemble in *vitro* and then delivered into the cells by a laser-assisted transfection method, wherein the cell is surrounded by a liquid and the laser focal point is in the liquid adjacent to the cell wall and not directly in contact with the cell wall and/or the plant cell.

The term "laser-assisted transfection method" includes a method for shuttling RNPs into intact plant cells of any organ and tissue of a plant, whereby the penetration of the RNPs is granted by indirect laser irradiation of the said plant cell. Indirect laser irradiation of an intact cell is achieved by focusing a laser beam, in particular the laser focal point by means of a magnifying lens such as an objective of a microscope in the adjacency of the cell and not directly onto the cell i.e. not directly hitting the plant cell, in particular the cell wall of the plant cell.

The term "laser focal point" pertains to a point in space defined by three-dimensional spatial coordinates wherein a laser beam is directed to after passing through a magnifying lens such as but not limited to an objective of a microscope. The laser focal point is affected by the features of the lens such as the curvature and the focal length of the lens and is found at the distance between the front of the lens and the point in space where the laser beam achieves its minimum in diameter. This point in space is the point where the laser is in focus. For the present disclosure, the laser focal point is the point in space where the laser is in focus and it is hitting the liquid medium surrounding the plant cell or plant explant.

The term "adjacency to" pertains to a lateral or vertical distance, i.e. aside, beneath or above the border of the plant cell(s) of 1 to 10 µm and more preferably 3 to 8 µm and even more preferably 2 to 5 µm either laterally or vertically to the plant cell(s), in particular to the plant cell wall. This is achieved for example by: i) layering or submerging a plant explant comprising a plurality of intact plant cells or a suspension of isolated intact plant cells into a liquid deposited onto a transparent support suitable for microscopy; ii) placing this support on a microscope table, iii) engaging an objective to visualize and magnify the plant cells; iv) putting the plant cell in focus, i.e. by obtaining a sharp image of the plant cell sample; and then v) by either selecting a region next to the plant cell in the range of values described above or by defocussing the cell in the vertical direction beneath or above the cell boundaries of the same range of values described above. Those skilled in the art can achieve the exact lateral or vertical displacement of the plant cell in focus by the range of values described above by using the x and y wheels of the microscope table for the lateral displacement or by using the focusing wheels of the microscope for the vertical displacement or if the microscope is equipped with a motorized stage, by changing the horizontal and vertical coordinates of the table by using the software driving the motorized table of the microscope.

The term "liquid" includes any liquid medium including water, any physiological solution including but not limited to physiological saline and any similar physiological solution, any cell culture liquid medium, any buffer including but not limited to phosphate buffer saline, Tris-based buffers, HEPES-based buffers or any other buffer known to those skilled in the art compatible with plant cells and suitable for any molecular biology work.

The RNP complex can be constituted e.g. of a Cas9 protein and a single guide RNA or the Cas9 protein, the guide RNA and the tracrRNA, wherein in particular the tracrRNA is labeled with a visual marker. The complex can be constituted by any other nuclease associated with the RNA determining the target specificity. To those skilled in the art it will be apparent that the RNP can be also constituted of a nuclease with a modified RNA molecule such as, but not limited to, a RNA molecule carrying base analogue substitutions or base modifications or substitutions of the phosphodiester bond or a visual marker like a fluorescent dye.

The intact plant cells comprising a cell wall can be obtained from cell suspensions of single cells or cell aggregates or from tissues such as anthers, callus, cotyledons, embryo, flowers, leaves, pods, roots, seeds and stems or can be part of an intact plant. In an advantageous embodiment, the plant cell is isolated from plant leaves, flowers, seeds, roots or cotyledons before providing the pre-assembled ribonucleoprotein (RNP)-complex.

The RNP complex is not restricted to nuclease activity. For example, a catalytically inactive version of the Cas9 protein is unable to cut DNA but it can still be recruited to specific DNA sequences by gRNAs. If it is expressed as a fusion protein with, e.g., a transcriptional activator or a cytidine deaminase, it can be delivered as RNP complex to e.g. activate transcription of a specific gene or mediate the direct conversion of cytidine to uridine.

The methods described in the present disclosure may be used to deliver other DNA-modifying enzymes including, but not limited to, Zinc Finger Nucleases (ZFN), Transcription Activator-Like Effectors (TALE), TALE nucleases (TALEN), methyl-transferases, histone deacethylases transcription factors and transcription repressors.

As used in the present disclosure, "cell", "cell line", and "cell culture" can be used interchangeably and all such designations include progeny. It is also understood that all progeny may not be precisely identical in DNA content, due to deliberate or inadvertent mutations. Mutant progeny that have the same functionality as screened for in the originally transformed cell are included.

As used herein, the phrase "coding sequence", "encoding sequence", "structural nucleotide sequence" or "structural nucleic acid molecule" refers to a nucleotide sequence that is translated into a polypeptide, usually via mRNA, when placed under the control of appropriate regulatory sequences. The boundaries of the coding sequence are determined by a translation start codon at the 5'-terminus and a translation stop codon at the 3'-terminus. A coding sequence can include, but is not limited to, genomic DNA, cDNA, EST and recombinant nucleotide sequences.

The term "gene" refers to a DNA sequence that comprises control and coding sequences necessary for the production of a recoverable bioactive polypeptide or precursor. Endogenous gene are those that originate from within an organism, tissue, or cell.

The term "isolated" describes any molecule separated from its natural source.
As used herein, the term "nucleic acid" refers to a single or double-stranded polymer of deoxyribonucleotide or ribonucleotide bases read from the 5' to the 3' end. The "nucleic acid" may also optionally contain non-naturally occurring or altered nucleotide bases that permit correct read through by a polymerase and do not reduce expression of a polypeptide encoded by that nucleic acid. The term "nucleotide sequence" or "nucleic acid sequence" refers to both the sense and antisense strands of a nucleic acid as either individual single strands or in the duplex. The term "ribonucleic acid" (RNA) is inclusive of RNAi (inhibitory RNA), dsRNA (double stranded RNA), siRNA (small interfering RNA), mRNA (messenger RNA), miRNA (micro-RNA), tRNA (transfer RNA, whether charged or discharged with a corresponding acylated amino acid), and cRNA (complementary RNA) and the term "deoxyribonucleic acid" (DNA) is inclusive of cDNA and genomic DNA and DNA-RNA hybrids. The words "nucleic acid segment", "nucleotide sequence segment", or more generally "segment" will be understood by those in the art as a functional term that includes both genomic sequences, ribosomal RNA sequences, transfer RNA sequences, messenger RNA sequences, operon sequences and smaller engineered nucleotide sequences that express or may be adapted to express, proteins, polypeptides or peptides.

As used herein, the term "intact cells" are characterized by an intact cell membrane and/or cell wall. In particular, if the intact cells are eukaryotic cells like plant cells, the intact plant cells comprise a cell wall. Preferably, an intact cell is collected from and/or comprised within a differentiated tissue.

As used in this disclosure "differentiated" and "undifferentiated" are relative terms depending on the context in which they are used. Specifically, in reference to a particular type of self-renewing stem cell, the term "undifferentiated" refers back to the same self-renewing stem cell, whereas the term "differentiated" refers to one or more of the relatively mature phenotypes the stem cell can generate - as discernable by morphological criteria, antigenic markers, and gene transcripts they produce. Undifferentiated pluripotent stem cells including iPS cells have the ability to differentiate into all three germ layers. The cells differentiated from them do not, and can readily be recognized by one skilled in the art by morphological criteria. In particular, differentiated plant cells include but are not limited to epidermal and mesophyll cells from cotyledons or leaves, epidermal or vascular cells from roots, epidermal or vascular cells from stems.

In some advantageous embodiments, intact plant cell in the methods according to the present disclosure is an intact differentiated plant cell.

The term " altering the genome" is used in the present disclosure for inducing one or more single or double stranded DNA breaks (DSB) in the cell genome, which results in a mutation that leads to / results in inhibiting expression of a target gene in said cell of which refers to the absence (or observable decrease) in the level of protein and/or mRNA product from the target gene.

The term "plant" includes the plant body, plant organs (for example, leaves, petals, stem, root, rhizome, and seeds), plant tissues (for example, epidermis, phloem, parenchyma, xylem, and vascular bundle), and plant cells. In addition, the term "plant cell" includes cell suspension cultures, embryos, meristematic tissue regions, callus tissues, cells derived from leaves and roots. When plant culture cells are transformed, an organ or individual is regenerated from the transformed cells by a known tissue culture method. These operations are readily performed by those skilled in the art. An example is described below. Firstly, the transformed plant cells are cultured in a sterilized callus forming medium (containing a carbon source, saccharides, nutrients, vitamins, inorganics, and phytohormones such as auxin and cytokinin), thereby forming a dedifferentiated callus which indefinitely proliferates (callus induction). The formed callus is transferred to a new medium containing a plant growth regulator such as auxin, and further proliferated thereon (subcultivation). When the callus induction is carried out on a solid medium such as agar and subcultivation is carried out in a liquid medium, the respective cultures are efficiently achieved. Secondly, the callus proliferated by subcultivation is cultured under appropriate conditions, thereby inducing re-differentiation of the organ (inductive re-differentiation), and regenerating the plant body. The inductive re-differentiation is achieved by appropriately adjusting the type and amount of the various components of the medium, including plant growth regulators such as auxin and cytokinin, and the carbon source, and the light and temperature. The inductive re-differentiation forms adventitious embryos, adventitious roots, adventitious buds, adventitious foliage, and others, and they are grown into a complete plant body. The plant before being a complete plant body may be stored in the form of, for example, capsulated artificial seeds, dry embryos, lyophilized cells, or tissues. The term "plant explant" as used herein includes any portion of a plant including a plurality of intact plant cells derived by any organ and/or tissue of an organ including but not limited to any seed, embryo, fruit, anther, ovary, leaf, stem, roots and any other tissue or cell type derived from a dicotyledonous or monocotyledonous plant species.

As used herein and mentioned above "genetic modification" or "altering the genome" means that a gene may be removed, or "knocked out", using a nucleic acid modifying protein like a nuclease. The present disclosure pertains to methods for editing eukaryotic genomes like mammalian or plant genomes using DNA-free strategies. Sequence-specific nucleases (including ZFNs, homing endonucleases, TAL-effector nucleases, CRISPR-associated systems [Cas9]) are introduced into plant cells in the form of a pre-assembled ribonucleoprotein (RNP)-complex comprising a nucleic acid modifying protein and a ribonucleic acid. The functional nucleases are targeted to specific sequences, and cut the cellular DNA at predetermined loci. The DNA damage triggers the plant cell to repair the double strand break. Mistakes (e.g., point mutations or small insertions/deletions) made during DNA repair then alter DNA sequences *in vivo.* As mentioned above, unlike conventional DNA transformation, the protein or RNA-based genome editing strategies described herein specifically modify target nucleic acid sequences at a high rate, i.e. the vast majority of mutations are found in the genome location defined by the sequence-specificity of the RNP. Since no exogenous DNA is introduced using these method, this process is termed DNA-free plant genome editing.

The nucleic acid modifying protein may be a sequence -specific nuclease like a TAL effector- nuclease, a homing endonuclease, a zinc finger nuclease (ZFN), or a CRISPR enzyme like a CRISPR-Cas9 endonuclease. The term "CRISPR enzymes", such as a Cas protein, include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Cscl, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of S. *pyogenes* Cas9 protein may be found in the SwissProt database under accession number Q99ZW2. In some embodiments, the unmodified CRISPR enzyme has DNA cleavage activity, such as Cas9. In some embodiments, the CRISPR enzyme directs cleavage of one or both strands at the location of a target sequence, such as within the target sequence and/or within the complement of the target sequence. In some advantageous embodiments, the nucleic acid modifying protein is a CRISPR-Cas9 endonuclease, a CRISPR-Cpf1 nuclease or a CRISPR-C2c2 endoribonuclease.

In some advantageous embodiments, the RNP-complex used in the methods of the present disclosure comprises a nucleic acid comprising a crRNA and a tracrRNA, or a chimeric cr/tracrRNA hybrid, wherein the crRNA and tracrRNA, or the cr/tracrRNA hybrid, is targeted to a sequence that is endogenous to the cell; and a Cas9 endonuclease molecule that induces a double strand break at or near the sequence to which the crRNA and tracrRNA sequence is targeted, or at or near the sequence to which the cr/tracrRNA hybrid is targeted. In some advantageous embodiment, the tracrRNA is labeled with a visual marker like a fluorescent marker like a fluorescent dye.

As used herein the term "endonuclease" refers to an enzyme capable of causing a single or double - stranded break in a DNA molecule.

As defined herein the term "exonuclease" refers to an enzyme that works by cleaving nucleotides one at a time from the end (exo) of a polynucleotide chain causing a hydrolyzing reaction that breaks phosphodiester bonds at either the 3' or the 5' to occur.

As used herein the term "sequence-specific nuclease" refers to any nuclease enzyme which is able to induce a double-strand DNA break at a desired and predetermined genomic locus

As used herein the term "vector" refers to a nucleic acid molecule capable of transporting another nucleic acid to which it has been linked into a cell, or a cell compartment.

As used herein the term "zinc finger nuclease" refers to artificial restriction enzymes generated by fusing a zinc finger DNA-binding domain to a DNA-cleavage domain. Briefly, ZFNs are synthetic proteins comprising an engineered zinc finger DNA-binding domain fused to the cleavage domain of the Fokl restriction endonuclease. ZFNs may be used to induce double-stranded breaks in specific DNA sequences and thereby promote site-specific homologous recombination and targeted manipulation of genomic sequences.

As used herein the term "TAL-effector endonuclease" refers to artificial restriction enzymes generated by fusing a DNA recognition domain deriving from TALE proteins of Xanthomonas to a catalytic domain of a nuclease, as described by Voytas and Bogdanove in WO 2011/072246. TAL-effector endonucleases are named TALEN^{™} by the applicant (Cellectis, 8 rue de la Croix Jarry, 75013 PARIS).

As used herein the term "visual marker" refers to molecules that are / can be detected optically due to their light absorbing and emitting properties, include but not limited to dyes, fluorescent dyes, fluorescent proteins, quantum dots. An enzyme that produces a dye or fluorescent dye or a pigment by converting an endogenous substrate or upon addition of a substrate is also considered a visual marker. Examples include e.g. β-glucuronidase, β-galactosidase, anthocyanidin synthase, luciferase, phytoene desaturase and others. In the context of this invention, a transcription factor or inducer of gene expression, that leads to the expression of an endogenous gene that subsequently causes a change in the optical properties of the cell(s) that have received the genome-modifying formulation, is also considered a visual marker. Examples are transcription factors from the MYC, MYB, and WD40 families, such as MdMYB1, MdMYB10 and MdMYBA from apples or Arabidopsis PAP1 (production of anthocyanin pigment 1). In advantageous embodiments, the ribonucleic acid of the pre-assembled ribonucleoprotein (RNP)-complex is fluorescent labeled e.g. with a fluorescent dye and/or a fluorescent protein. An example for such a visual marker is ATTO 550 (ATTO-TEC GmbH), a fluorescent label related to the well-known dyes Rhodamine 6G and Rhodamine B. Characteristic features of the label are strong absorption, high fluorescence quantum yield, and high thermal and photo-stability. The dye is moderately hydrophilic. ATTO 550 is a cationic dye. After coupling to a substrate the dye carries a net electrical charge of +1. As supplied ATTO 550 consists of three isomers with practically identical absorption and fluorescence. The fluorescence is excited most efficiently in the range 540 - 565 nm.

As used herein the term "different RNP complexes" indicates that said RNP complexes differ structurally in at least one component. This component can be the nucleic acid modifying protein or the Cas9 endonuclease molecule or the tracrRNA or the crRNA or any combination thereof.

In some advantageous embodiments, the methods according to the present disclosure comprise a multiplex genome modifying formulation (multiplexing) for introducing two RNP-complexes simultaneously in e.g. leaf cells (as described in example 4, 6 and 9). Furthermore, in some advantageous embodiments the methods according to the present disclosure comprise a multiplexing with two physical delivery methods (particle bombardment and celite-503 abrasion, see examples 4 and 9).

Therefore, in some advantageous embodiments the genome-modifying formulation comprises at least two different pre-assembled ribonucleoprotein (RNP)-complexes, in particular wherein the two different pre-assembled ribonucleoprotein (RNP)-complexes target different target sequences in the genome. Furthermore, in some advantageous embodiments at least two different genome-modifying formulation are provided for delivering different RNP-complexes into the cell etc.

As used herein the term "multiplexing" refers to a genome-modifying formulation comprising a mixture of at least two structurally different RNP complexes. The structurally different RNP complexes can be pre-assembled individually and then added together. The structurally different RNP complexes can also be prepared by adding the components in any other sequence, e.g. to generate structurally different RNP complexes in a combinatorial manner.

In some advantageous embodiments, the RNP-complex used in the methods of the present disclosure comprises a nucleic acid comprising a crRNA and a tracrRNA, or a chimeric cr/tracrRNA hybrid, wherein the crRNA and tracrRNA, or the cr/tracrRNA hybrid, is targeted to a sequence that is endogenous to the cell; and a Cas9 endonuclease molecule that induces a double strand break at or near the sequence to which the crRNA and tracrRNA sequence is targeted, or at or near the sequence to which the cr/tracrRNA hybrid is targeted.

In one aspect, the present disclosure pertains to method for targeted genetic modification of a plant genome without inserting exogenous genetic material. The method can include (i) providing a plant cell that contains an endogenous gene to be modified, (ii) providing a genome-modifying formulation comprising a pre-assembled ribonucleoprotein (RNP)-complex comprising a nucleic acid modifying protein and a ribonucleic acid, (iii) delivering said RNP-complex into the cell by a laser-assisted transfection method to get past the cell wall, and (iv) inducing one or more single or double stranded DNA breaks (DSB) in the genome, to produce a plant cell or cells having a detectable targeted genomic modification without the presence of any exogenous genetic material in the plant genome. The DSB can be repaired by non-homologous end joining (NHEJ).

The methods and compositions of the present disclosure may be applied to an intact differentiated cell like a plant cell, an animal cell, a human cell and a microbial cell. In particular, the cell may be comprised in a tissue and/or an organism. In some advantageous embodiments, the plant species used in the methods provided herein belong to the *Solanaceae* family and is *Nicotiana benthamiana or N. tabacum,* although in a further aspect the plant species may be any monocot or dicot plant or algae, such as (without limitation) *Arabidopsis thaliana;* field crops (e.g., alfalfa, barley, bean, corn, cotton, flax, pea, rape, rice, rye, safflower, sorghum, soybean, sunflower, tobacco, and wheat); vegetable crops (e.g., asparagus, beet, broccoli, cabbage, carrot, cauliflower, celery, cucumber, eggplant, lettuce, onion, pepper, potato, pumpkin, radish, spinach, squash, taro, tomato, and zucchini); fruit and nut crops (e.g., almond, apple, apricot, banana, blackberry, blueberry, cacao, cherry, coconut, cranberry, date, fajoa, filbert, grape, grapefruit, guava, kiwi, lemon, lime, mango, melon, nectarine, orange, papaya, passion fruit, peach, peanut, pear, pineapple, pistachio, plum, raspberry, strawberry, tangerine, walnut, and watermelon); and ornamentals (e.g., alder, ash, aspen, azalea, birch, boxwood, camellia, carnation, chrysanthemum, elm, fir, ivy, jasmine, juniper, oak, palm, poplar, pine, redwood, rhododendron, rose, and rubber).

In some advantageous embodiments, the intact differentiated plant cell is isolated from plant leaves, flowers, roots or cotyledons before providing the pre-assembled ribonucleoprotein (RNP)-complex.

In some advantageous embodiments, the pre-assembled ribonucleoprotein (RNP)-complex is co-delivered with one or more components, including but not limited to dNTP, ddNTP, non-natural base analogues, di-nucleotides, trinucleotides, oligonucleotides , enzyme inhibitors, sugars, amino acids, proteins, antibodies, transcription factors and other DNA-binding proteins, ribonucleic acids in the form of mRNA, siRNA, miRNA.

Therefore, in some advantageous embodiments, the present disclosure pertains to methods for altering the genome of an intact differentiated cell(s) without inserting exogenous genetic material into the genome comprising:
(i) providing an intact differentiated plant cell that comprises an endogenous gene to be modified;
(ii) providing a genome-modifying formulation comprising a pre-assembled ribonucleoprotein (RNP)-complex comprising a nucleic acid modifying protein and ribonucleic acid, wherein the ribonucleic acid is labeled with a visual marker;
(iii) delivering said RNP-complex into the cell by a laser-assisted transfection method, wherein the cell is surrounded by a liquid and the laser focal point is in the liquid adjacent to the cell wall and not directly in contact with the cell wall;
(iv) inducing one or more single or double stranded DNA breaks in the cell genome to produce a cell having a detectable targeted genomic modification without the presence of any exogenous genetic material in the cell genome.

Based on these findings, the inventors developed a novel method for the generation of DNA free genome-edited plants through the transfection of intact plant cells with CRISPR/Cas9 RNPs. For example, a high-powered multiphoton laser may be focused 2-5 µm below a leaf sample comprising intact plant cells and briefly irradiated the sample at a laser power of ^{∼}2 W. The laser power may be kept at 70%. In addition, the offset, Gain and EOM of the laser pulsing controller maybe kept at 0.00.

Pulsed operation of lasers refers to any laser not classified as continuous wave, so that the optical power appears in pulses of some duration at some repetition rate. Other laser like Nd:YAG laser was also used to create a pulse energy varied between 260 to 335 µJ. Continuous waves also can be used with a λ = 975 nm, laser power of 0.7kW/cm² (Padilla-Martinez et al., 2014). The mentioned laser-associated transfection method can be used for the direct delivery of RNPs into the intact cells of e.g. tobacco leaf discs, which are easy to prepare and handle, thus avoiding the laborious preparation of protoplasts or zygotes. A preassembled RNP comprising the Cas9 protein, crRNA and ATTO-550-labeled tracrRNA targeting the tobacco pds or actin depolymerizing factor (*adf*) genes is introduced in the leaf cells. The fluorescent tracrRNA allows the direct screening of transfected cells and makes the use of a selectable marker gene unnecessary. Nevertheless, RNP-mediated genome editing is generally laborious because the absence of a selectable marker gene still requires a time-consuming screening process in order to identify rare mutants in a large background of wild-type cells.

In some advantageous embodiments, the methods according to the present disclosure comprise the further step of identifying the cells transfected with the RNP-complex by microscopy, in particular by fluorescence microscopy when using a fluorescent visual marker labeled to the RNA of the RNP-complex.

In another embodiment, the methods according to the present disclosure comprise further the step of selecting the cells transfected with the RNP-complex and optionally regenerating the cells to intact plantlets by growing in and/or on a growth media. For example, the selected cells comprising the RNP-complex can be used for the regeneration of cell lines, whole tissues or organisms.

Therefore, an advantageous embodiment of the present disclosure pertains to a method for altering the genome of an intact plant cell without inserting exogenous genetic material into the genome comprising:
(i) providing an intact plant cell that comprises an endogenous gene to be modified;
(ii) providing a genome-modifying formulation comprising a pre-assembled ribonucleoprotein (RNP)-complex comprising a nucleic acid-modifying protein and ribonucleic acid, wherein the ribonucleic acid is labeled with a visual marker;
(iii) delivering said RNP-complex into the plant cell with a laser-assisted transfection method, wherein the cell is surrounded by a liquid and the laser focal point is in the liquid adjacent to the cell wall and not directly in contact with the cell wall;
(iv) inducing one or more single or double stranded DNA breaks in the cell genome to produce a cell having a detectable targeted genomic modification without the presence of any exogenous genetic material in the cell genome.
(v) identifying the cells transfected with the RNP-complex by microscopy, in particular by fluorescence microscopy.
(vi) selecting the cells transfected with the RNP-complex and optionally regenerating the cells to intact plantlets by growing in and/or on a growth media.

Plant tissue and cell culture growth media according to the present disclosure are generally made up of some or all of the following components: macronutrients, micronutrients, vitamins, amino acids or other nitrogen supplements, sugar(s), other undefined organic supplements, solidifying agents or support systems, and growth regulators. Several media formulations are commonly used for the majority of all cell and tissue culture work, e.g. Banana Medium, BM Medium, CHU (N6) Medium, Gamborg B5 Medium, Linsmaier & Skoog Medium, Murashige & Skoog Medium (MS), Modified MS Medium, Nitsch Medium, Orchid Medium, Schenk & Hildebrandt Medium.

A further advantageous embodiment of the present disclosure pertains to a method to produce genome-edited plants without inserting exogenous genetic material into the genome of the plant comprising the steps of:
(i) providing a plant explant isolated by any organ and tissue of a plant and comprising intact plant cells comprising an endogenous gene to be modified;
(ii) providing a genome-modifying formulation comprising a pre-assembled ribonucleoprotein (RNP)-complex comprising a nucleic acid-modifying protein and ribonucleic acid, wherein the ribonucleic acid is labeled with a visual marker;
(iii) layering the plant explant onto the RNP-complex formulation in a transparent support;
(iv) delivering said RNP-complex into a plant cell of the plant explant with laser-assisted transfection, wherein the cell is surrounded by a liquid and the laser focal point is in the liquid adjacent to the cell wall and not directly in contact with the cell wall;
(v) inducing one or more single or double stranded DNA breaks in the cell genome to produce a cell having a detectable targeted genomic modification without the presence of any exogenous genetic material in the cell genome.
(vi) identifying the cells transfected with the RNP-complex by microscopy, in particular by fluorescence microscopy;
(vii) selecting the cells transfected with the RNP-complex from the plant explant
(viii) regenerating the cells to intact plantlets by growing in and/or on a growth media.

In some embodiments, the method comprises the further step of:
(ix) analyzing the plantlets to confirm editing of the target gene using any method for genetic characterization of genome-edited plants such as high-resolution melt analysis for identifications of mutated alleles;
(x) selecting the analyzed plantlets scoring positive and further growing those plantlets to full plants.

A "transparent support" includes any support that can transmit light of different wavelength spanning the visible spectrum and beyond, i.e. in the UV range and/or in the near infrared, infrared and far-infrared spectrum. A transparent support includes but it is not limited to any glass support, any quartz support, any borosilicate support or any optical grade plastic support including but not limited to any polyethylene, polystyrene, cyclic oleofin polymers and other polymers made supports of any thickness as known to those skilled in the art.

A "method for genetic characterization of a genome edited plant" includes any method to obtain information on the DNA sequence of one or more gene(s) and/or allele(s) thereof, with the scope to confirm that the plant derived by the genome editing method of the present invention has been successfully modified for the particular target gene or genes. For the methods according to the present disclosure the genetic characterization is carried out by using any technique known to those skilled in the art including but not limited to Sanger sequencing, any next generation sequencing approach including but not limited to pyrosequencing, SOLiD sequencing, sequencing by synthesis (SBS) or any other sequencing method knows to those skilled in the art or any other method suitable to derive information on the allelic composition of the genome edited plant including but not limited to high resolution melt analysis (HRMA).

In some examples of the present description, the ATTO-550-labeled tracrRNA allowed the rapid identification of transfected cells by fluorescence microscopy. The RNP-containing cells are screened 48 h after the laser-assisted transfection, identified and selected RNP-containing cells are excised at small leaf tissue fragments of different sizes (^{∼}0.1-0.3 cm radius) depending on the intensity and distribution of the RNP signal. The selected leaf fragments are grown on MS medium to regenerate intact plantlets. Following regeneration, a simple PCR-based HRMA procedure can be used to identify the mutants because this method has sufficient resolution to detect single-nucleotide changes [Hidalgo-Grass et al., 2010; Denbow et al., 2018; Li et al., 2018]. In this manner, it is possible to screen 96 plants in 2 h, a much higher throughput than it could be achieved using e.g. the T7 assay for detection and selection of mutants [EP3392339]. A total of 1673 plants in the pds gene editing experiment were analyzed and 147 homozygous or biallelic mutants and 442 heterozygous mutants were identified. The homozygous or biallelic mutants were characterized in more detail by Sanger sequencing across the target site, revealing 93 homozygous and 54 biallelic mutants and confirming the positive HRMA results.

Therefore, with the novel method according to the present disclosure for the development and screening of DNA free genome-edited plants, an overall mutation efficiency of 8.7% in the PDS gene within a very short time period (less than 8 months) was achieved. Furthermore, 1011 plants in the *adf* gene editing experiment were analyzed by HRMA and 57 homozygous or biallelic mutants and 110 heterozygous mutants were identified. In the former group, Sanger sequencing confirmed the positive HRMA results and resolved 48 homozygous mutants and 9 biallelic mutants.

In addition, the composition comprising the RNP-complexes can also contain enzymatic functions such as cellulases, pectinases, lipases, collagenases etc. and / or specific ligands as for example to facilitate attachment to cell walls, cell membranes, extracellular receptors.

The RNP-complexes and / or the particulate components can further comprise a fluorescent dye and / or light absorbing structure and / or component with a refractive index match to enable the use of optical tweezers (originally called "single-beam gradient force trap").

In some advantageous embodiments, the intact cell(s) is obtained from a non-sterile environment and/or provided under a non-sterile environment, e.g. a plant that is grown in a green house, a growth chamber, a field, a habitat, or a natural environment.

In some advantageous embodiments the intact cell(s) are further cultivated in a non-sterile environment after the pre-assembled ribonucleoprotein (RNP)-complex has been delivered to said intact cells(s). Such non-sterile environments may e.g. comprise a laboratory, a room, a green house, a growth chamber, a growth cabinet, a field, a garden, or a natural environment.

In some advantageous embodiments, the intact differentiated cell(s) are exposed to stress condition before and/or while delivering the pre-assembled ribonucleoprotein (RNP)-complex, including but not limited to UV-stress, heat-stress, cold-stress, draught, reactive oxygen-species or chemicals.

In further embodiments, the genome-modifying formulation, in particular the (RNP)-complex does not comprise DNA. Direct delivery of RNP complexes for genome editing is highly desirable because it does not involve DNA, precluding the possibility of the resulting plant to be transgenic.

In particular, the genome-modifying formulation, in particular the (RNP)-complex may not comprise a marker gene and/or beside the visual marker another selectable marker. A "marker" or "selectable marker" may be a detectable genetic trait or segment of DNA that can be identified and tracked. A marker gene typically serves as a flag for another gene, sometimes called the target gene. A marker gene is typically used with a target gene being used to transform target cells. Target cells that heritably receive the target gene can be identified by selecting for cells that also express the marker gene. The marker gene is near enough to the target gene so that the two genes (the marker gene and the target gene) are genetically linked and are usually inherited together.

In some advantageous embodiments, the genome-modifying formulation comprising a plurality of different pre-assembled ribonucleoprotein (RNP)-complex, in particular the ribonucleoprotein (RNP)-complex differ in the nucleic acid sequence of the ribonucleic acid comprised in the complex. Therefore, the genome-modifying formulation comprises a plurality of pre-assembled ribonucleoprotein (RNP)-complexes, in particular with different ribonucleoprotein (RNP)-complexes. In some embodiments, the different ribonucleoprotein (RNP)-complexes can (A) be mixed and coated onto the same particle or they can (B) be coated separately onto the particles and then the coated particles could be mixed. Scenario (A) is more useful for the "targeted" approach and standard applications where an individual particle (=carrier) delivers a plurality of RNP-complexes to a single cell.

In some advantageous embodiments, the intact differentiated cell is exposed to the pre-assembled ribonucleoprotein (RNP)-complex, in particular in the presence of a carrier material, wherein after delivering said RNP-complex into the cell, the genome of the cell is altered/modified, and wherein after the modification/alteration the cells are further cultivated to derive a cell, cell line, tissue or organism with a modified (altered) genome without the presence of a marker gene.

As mentioned above, a plant cell according to the present disclosure comprises a cell wall, in particular said plant cell is from a plant from the *Solanaceae* family, like *Nicotiana tabacum* and *Nicotiana benthamiana* from the genus *Nicotiana.*

Furthermore, the nucleic acid modifying protein according to the present disclosure may be a CRISPR-Cas9 endonuclease, a CRISPR-Cpf1 nuclease or a CRISPR-C2c2 endoribonuclease or any other designer nuclease and in particular the ribonucleic acid comprises a crRNA and a tracrRNA, or a chimeric cr/tracrRNA hybrid. In some advantageous embodiments, the RNP-complex comprises a crRNA, a tracrRNA and a CRISPR-Cas9 endonuclease that target to a DNA sequence that is endogenous to the cell and that induces a double strand break at or near the sequence to which the crRNA and tracrRNA sequence is targeted to, or at or near the sequence to which the cr/tracrRNA is targeted to.

In some further embodiments, the plant cell is exposed to the pre-assembled ribonucleoprotein (RNP)-complex, wherein after delivering said RNP-complex into the cell, the genome of the cell is altered/modified, and wherein after the modification/alteration the cells are further cultivated to derive a cell, cell line, tissue or organism with a modified (altered) genome without the presence of a marker gene.

In some further embodiments, the laser is a multiphoton laser operating under pulsing conditions and wherein the laser power is between 0.5 W and 3 W, preferably about 2 W, more preferably wherein the laser power is between 65 and 90%, preferably at 70% of 2 W and wherein preferably the wavelength is between 700 nm und 900 nm, preferably 800 nm.

As mentioned above, in some embodiments of the present disclosure the genome-modifying formulation comprises a plurality of different pre-assembled ribonucleoprotein (RNP)-complexes, in particular wherein the ribonucleoprotein (RNP)-complexes differ in the nucleic acid sequence of the ribonucleic acid comprised in the complexes. In particular, the genome-modifying formulation comprises at least two different pre-assembled ribonucleoprotein (RNP)-complexes, in particular wherein the two different pre-assembled ribonucleoprotein (RNP)-complexes target different target sequences in the genome.

### Methods and Examples

In the following examples, materials and methods of the present disclosure are provided. It should be understood that these examples are for illustrative purpose only and are not to be construed as limiting this disclosure in any manner. All publications, patents, and patent applications cited herein are hereby incorporated by reference in their entirety for all purposes.

### Example 1: Laser-assisted transfection introduction of DsRed protein into N. tabacum leaves

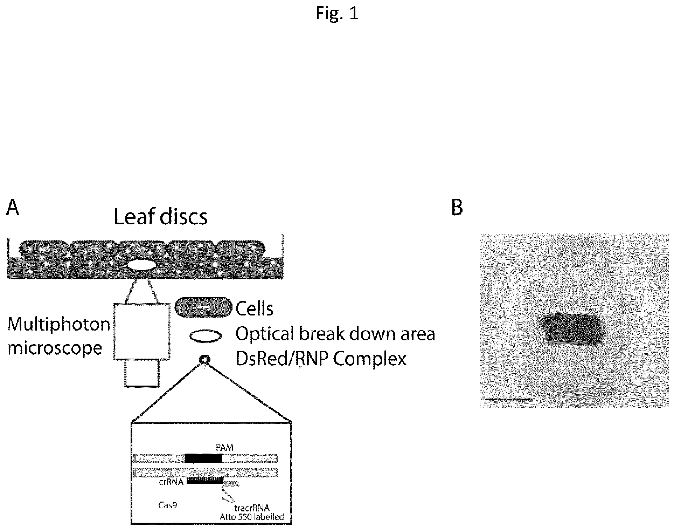

To test the delivery of proteins into cells through laser-assisted transfection method we used recombinant DsRed protein (*Discosoma* sp.red fluorescent protein gene; R2G mutant; Sack et al., 2015) for the initial experiments. In order to standardize the transfection conditions, wild-type tobacco plants (3-4 months old, when the plants had reached the 4-6 leaf stage) was used. The leaf discs (^{∼}10 x 5 mm) were placed in a microscopy-grade 35-mm petri dish with a glass bottom (ibidi µ-dish) containing 20 µL 0.25 µg/µL DsRed. The mid-rib was removed and the remaining leaf area was cut into approximately 10 mm x 5 mm size (Figure 1B). After several irradiation tests with the laser we identified conditions that promoted a local transient increase in membrane permeability without affecting cell viability. For all experiments, with our confocal system this was achieved by setting the laser power between 0.5 and 3W more preferably to ^{∼}2 W and by tuning the laser at a wavelength between 700 and 1200 nm more preferably at a wavelength of 900 nm. Moreover, by using the controller integrated in the software of the confocal system we further adjusted the laser power between at least 65 and 90% and more preferably at 70% of the nominal power of 2 W indicated above. In addition, the offset, gain and electro-optical modulator (EOM) settings of the laser controlling mask of the microscope software were both set to 0.00. The laser laser focal point was focused at 2-5 µm below the leaf sample and the plant tissue was irradiated by a short laser pulse focused to a very restricted region of the leaf sample targeting an area of approx. 2-3 cells. For this purpose we used a 40x water immersion objective. However, objectives of lower or higher magnification (e.g. a 20x or a 60x objectives) with dry or immersion lenses are also suitable for laser irradiation after fine-tuning the power settings. The resulting used laser-assisted transfection method transiently increased the permeability of the plasma membrane, allowing the uptake of DsRed into the leaf cells.

### Example 2: Identification of DsRed protein by fluorescence microscopy

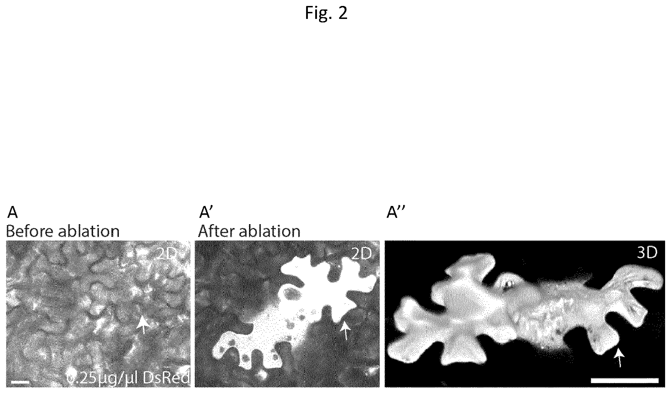

To confirm penetration of DsRed in individual cells leaf explant cells were analyzed before and after the laser pulse by confocal microscopy (Figure 2A and A'). A 3D-reconstruction of the transfected cells confirmed that DsRed was homogenously distributed in the cytoplasm (Figure 2A") and not merely distributed on the tissue surface. The introduction of DsRed protein into the leaves using the laser-assisted transfection method according to the present disclosure was also confirmed by visually inspecting the leaf discs under an Olympus X71 inverted fluorescence microscope using a fluorescence filter with an excitation peak of approximately 558 nm and an emission peak of approximately 583 nm for the detection of DsRed fluorescence.

### Example 3: Laser-assisted transfection method introduction of DsRed protein into Z. mays leaves

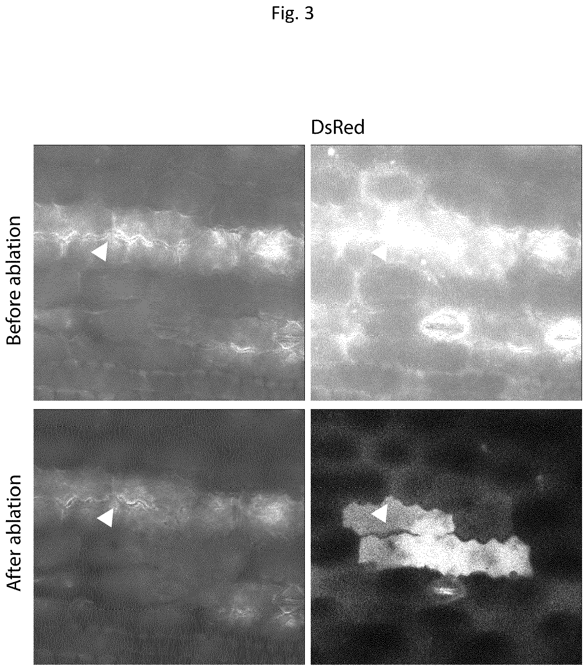

To further confirm the delivery of proteins into cells through the laser-assisted transfection method according to the present disclosure, the inventors used recombinant DsRed protein and explant obtained from 1-month-old Z. *mays* leaves, a monocotyledon crops. The experiment set up was as like example 1 and after the introduction of DsRed protein into the cells we identified the fluorescence as like example 2 (Figure 3).

### Example 4: Designing of crRNA

Genomic DNA was extracted from wild-type tobacco plants (3-4 months old, when the plants had reached the 4-6 leaf stage) [Pospíšilová et al., 1998] using the NucleoSpin Plant II kit (Macherey & Nagel). The target regions of the selected pds and *adf* genes were amplified from genomic DNA using a high-fidelity PCR system (Q5 high-fidelity DNA polymerase). The PCR products were purified from agarose gels using the NucleoSpin gel and PCR clean-up kit (Macherey & Nagel) and sequenced using the Sanger method prior to gRNA design, using the primers listed in the table 1. The gRNA sequences were designed using the Crispr RGEN Tools, Cas-Designer and CRISPR-P v2.0 online. The gRNA targeting the pds gene was 5'-TTT TTT TGG AAT ATC AGG TTT GG-3' and the gRNA targeting the *adf* gene was 5'-CTT GGA GCT GAA GAG GAA GAA GG-3'. BLAST analysis was used to identify any potential off-targets in the crRNA sequences.

**Table 1:**

| **Gene** | **Primer sequence** |
|---|---|
| ***adf*** | **5'-ATGTCTTTCAGATTCAGAGGG-3'** |
| | **5'-TCAGTGAG CGCG GTCTTT-3'** |
| ***pds*** | **5'-CTTGATTTTGTGGGTGAAGGA-3'** |
| | **5'-GCAAGGCAGAATACAGATCG-3'** |

### Example 5: Preparation of RNP complex

To prepare RNP complexes, we used crRNA, tracrRNA labeled with ATTO-550 and Cas9 protein synthesized by Integrated DNA Technologies, Inc. (IDT). We used crRNA-XT for all experiments, which has additional chemical modifications to optimize stability and performance. We mixed the crRNA and tracrRNA in equimolar concentrations. For all the experiments we used 100 µM concentration of crRNA and ATTO 550 labelled tracrRNA. For preparation of the RNP complex we initially heated the crRNA and ATTO 550 labelled tracrRNA 100 µM each at 95°C for 5 minutes. Further 120 pmol from the gRNA mix, 104 pmol Cas9 protein and 2.1 µl of PBS were added to make a final volume of 5 µL of RNP complex and kept at room temperature for 20 minutes.

### Example 6: Targeting endogenous pds gene through delivery of RNP complex into N. tabacum intact leaf cells using the laser-assisted transfection

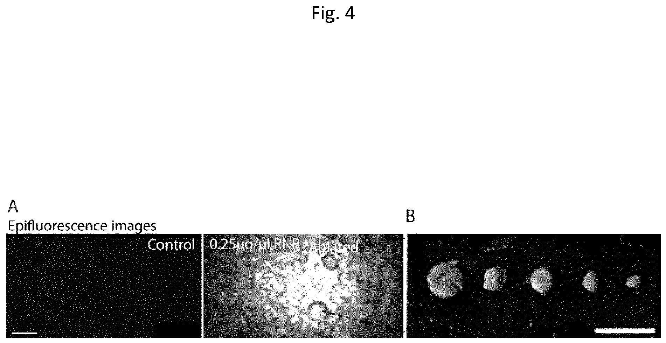

The crRNA was designed as like example 4 and the RNP complex was prepared as like example 5. Immediately after the preparation of RNP complex it was introduced into 3-4 months old *N. tabacum* intact leaf discs, which are easy to prepare and handle, thus avoiding the laborious preparation of protoplasts or zygotes. After the introduction of RNP the leaves were kept on MS medium (4.4 g/L MS-salts with vitamins (Duchefa M0222), 20 g/L sucrose, 0.6 mg/L thiamine-HCI, 7 g/L agar, pH 5.8) for 48 h. The leaf discs were visually inspected under an Olympus X71 inverted fluorescence microscope with an excitation peak of approximately 558 nm and an emission peak of approximately 583 nm for the detection of ATTO 550, 48 h post-transfection (Figure 4A). Regions showing fluorescence were excised using pipette tips (1 mL and 200 µL capacity, shortened with scissors to achieve a radius of 0.1-0.3 cm) according to the area of RNP fluorescence (Figure 4B). The selected regions containing the pds RNPs were transferred to MS medium (4.4 g/L MS-salts with vitamins (Duchefa M0222), 20 g/L sucrose, 0.6 mg/L thiamine-HCI, 7 g/L agar, pH 5.8) with hormones (1 mg/L 6-BAP, 0.1 mg/L NAA) and kept at 20 - 230C. The regenerated tissue was subcultured onto plates with fresh medium every 2 weeks until shoots appeared. All plants were regenerated without selection reagents such as antibiotics.

### Example 7: Targeting endogenous adf gene through delivery of RNP complex into N. tabacum intact leaf cells using the laser-assisted transfection method

The crRNA was designed as like example 4 and the RNP complex was prepared as like example 5. The endogenous *adf* gene was targeted as like example 5 using the corresponding gRNA. After the introduction of RNP, the tissue was allowed to regenerate onto plates with fresh medium, which was described in example 6 every 2 weeks until shoots appeared. The plantlets were then transferred onto MS medium without hormones and incubated at 20-25°C with a 16-h photoperiod (7000 lux) to induce root formation. *Adf* plants with roots were transferred to ED73 standard soil (Patzer) with 0-30% (v/v) sand, and grown in the greenhouse with a 16-h photoperiod (10000 lux, plus sunlight) at 70-90% humidity. All plants were regenerated without selection reagents such as antibiotics.

### Example 8: Analysis of pds gene phenotype

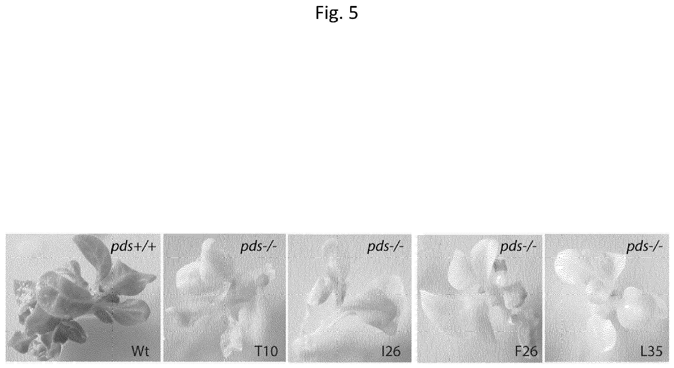

After the regeneration of the shoots, the inventors visually identified the albino phenotypes resulting for homozygous mutation of *pds* gene. Representative images of homozygous pds mutant plants with the anticipated albino phenotypes (homozygous lines F19, T10, L35 and 126) are provided in Figure 5. By selecting the region containing the cells transfected with RNPs, the inventors were able to avoid the inclusion of large numbers of wild-type cells to facilitate the screening and regeneration of mutants.

### Example 9: Analysis of pds gene mutation using high resolution melt analysis (HRMA) and confirmation using Sanger sequencing

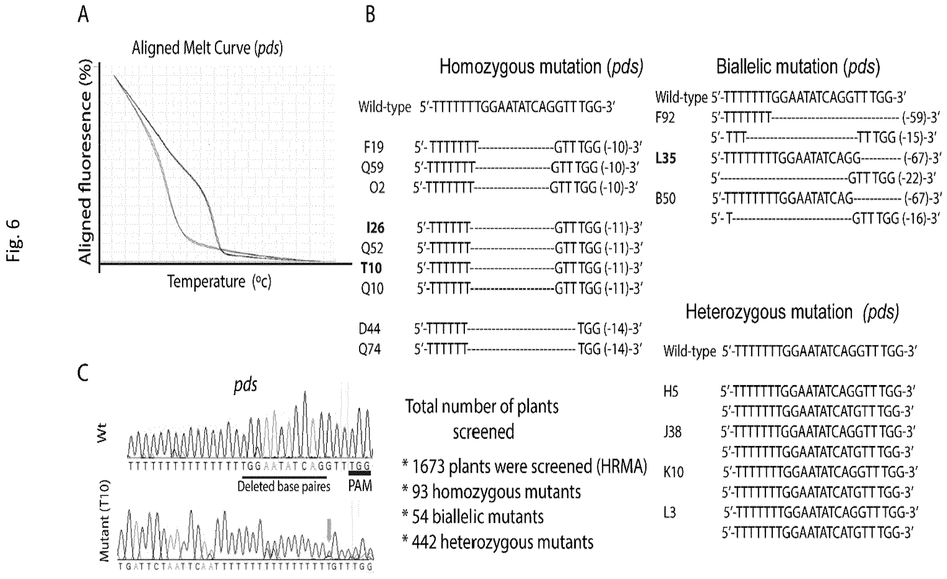

Following regeneration, the inventors used a simple PCR-based HRMA procedure to identify the mutants because this method has sufficient resolution to detect single-nucleotide changes [Hidalgo-Grass C and Strahilevitz J, 2010; Denbow et al., 2018; Li et al., 2018]. HRMA is also simpler, more sensitive, more specific, less expensive and quicker than other screening methods [Hung et al., 2008; Kennerson et al., 2007; McKinney et al. 2004; Willmore et al., 2004; Zhou et al., 2004]. In this manner, the inventors were able to screen 96 plants in 2 h, a much higher throughput than previously achieved using the T7 assay for detection and selection of mutants [Bortesi et al., 2017]. A total of 1673 plants were analyzed in the *pds* gene editing experiment and identified 147 homozygous/biallelic mutants and 442 heterozygous mutants. The melting curve of *pds* homozygous mutant T10 is compared to the wild-type control in Figure 6A. The primers used for HRMA is given in table 2. The homozygous/biallelic mutants were characterized in more detail by Sanger sequencing across the target site, revealing 93 homozygous and 54 biallelic mutants and confirming the positive HRMA results (Figure 6B). The Sanger sequencing primers are given in table 2. A representative chromatogram peak analysis is provided for line T10 in Figure 6C, showing a large deletion upstream of the PAM signal. With the laser-assisted transfection method according to the present disclosure, the inventors achieved an overall mutation efficiency of 8.7% in the *pds* gene within a very short time period (approximately 7-8 months).

**Table 2:**

| **Gene** | **Primer sequence** |
|---|---|
| ***pds* HRM** | **5'-ATCTGGCTGATGCTGGTCAC-3'** |
| | **5'-AAGGAATAAAATTAAAGGAAAGCATG-3'** |
| ***pds* Seq** | **5'-CCATTGACCGGTTAGCAGTT-3'** |
| | **5'-TGAACACCCTTGCAATTGTTTGAG-3'** |

### Example 10: Analysis of adf gene mutation using high resolution melt analysis and confirmation using Sanger sequencing

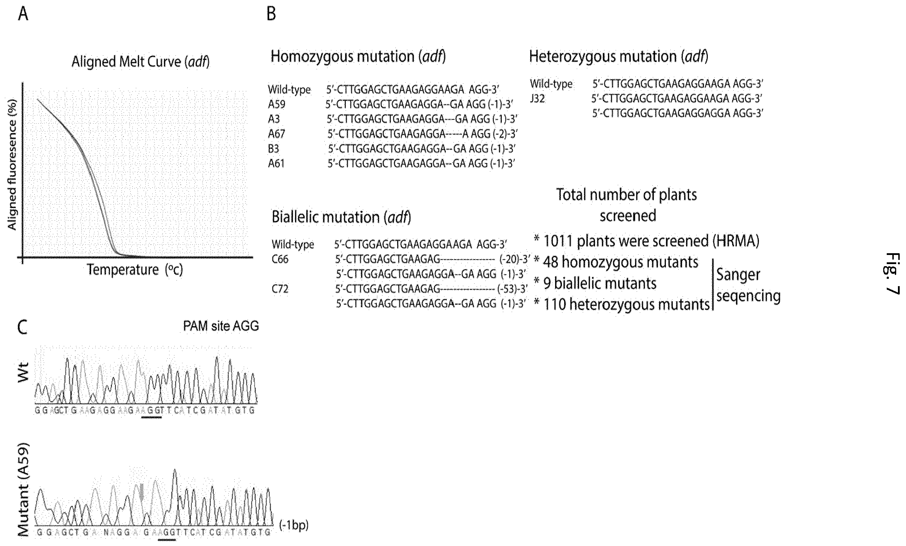

1011 plants were analyzed by HRMA in the *adf* gene editing experiment and identified 57 homozygous/biallelic mutants and 110 heterozygous mutants. The primer sequences used for HRMA are given in table 3. In the former group, Sanger sequencing confirmed the positive HRMA results and resolved 48 homozygous mutants and 9 biallelic mutants. The Sanger sequencing primers are given in table 3. The melting curve of *adf* homozygous mutant A59 is compared to the wild-type control in Figure 7A. Representative results for the homozygous, biallelic and heterozygous mutants are provided in Figure 7B. Sanger sequencing specified the nature of the mutations (a representative chromatogram for homozygous mutant A59 is provided in Figure 7C and shows a single-base deletion at the PAM site).

**Table 3:**

| **Gene** | **Primer sequence** |
|---|---|
| ***adf* HRM** | **5'-TTCTGGCATGGGTGTAGCTG-3'** |
| | **5'-GCTGCCAGTTTTCTCAACAA-3'** |
| ***adf* Seq** | **5'-AGTCAAGGTGCCTGCAATTTA-3'** |
| | **5'-CAAAGAAAATCTTGCTCTTTTGG-3'** |

### Example 11: Further analysis of the adf mutants

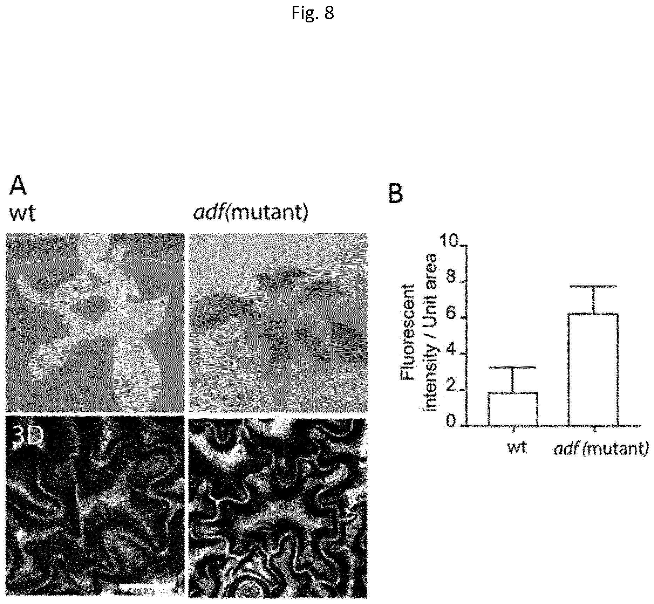

For further confirmation, the inventors stained T0 *adf* mutant plants with phalloidin to visualize the anticipated enhanced formation of actin filaments [Augustine et al., 2015]. The principal components of the actin cytoskeleton include monomeric actin (G-actin) and filamentous actin (F-actin), with G-actin being the dominant form [Nan et al., 2017; Hugo et al., 2017]. The role of *adf* is to depolymerize F-actin to G-actin. Thus, a mutation in the *adf* gene should promote the formation of more F-actin. Accordingly, confocal microscopy confirmed the presence of more actin filaments in the leaves of T0 *adf* mutant plants compared to wild-type plants (Figure 8A) based on image analysis of the actin fluorescence intensity normalized by the area of the stained actin using ImageJ (Figure 8B). Representative images of the wild-type and *adf* mutant plantlets (line A59) 2-3 months after transfection with RNP (Figure 8A) and of adult mutant T0 plants grown in the greenhouse showed that the *adf* mutation did not have a significant effect on the morphological phenotype.

### References

Augustine SM, Cherian AV, Syamaladevei DP and Subramonian N (2015) Erianthus arundinaceus HSP70 (EaHSP70) acts as a key regulator in the formation of anisotropic interdigitation in sugarcane (Saccharum spp. hybrid) in response to drought stress. Plant and Cell Physiology 56:2368-80.
Bortesi, L., Augustine, S.M., Fischer, R., Sack, M., and Zischewski, J. (2017). EU3392339. Improved genome editing in plant cells. (Patent application no. EP 17166753.8.).
Denbow, C., Ehivet, S. C. and Okumoto, S. (2018). High Resolution Melting Temperature Analysis to Identify CRISPR/Cas9 Mutants from Arabidopsis. Bio Protoc 8(14): e2944.
Hidalgo-Grass C and Strahilevitz J (2010) High-Resolution Melt Curve Analysis for Identification of Single Nucleotide Mutations in the Quinolone Resistance Gene aac(6')-Ib-cr∇. Antimicrobial Agents and Chemotherapy. 54(8): 3509-3511.
Hugo Wioland, Berengere Guichard, Yosuke Senju, Sarah Myram, Pekka Lappalainen, Antoine Jegou, Guillaume Romet-Lemonne. ADF/Cofilin accelerates actin dynamics by severing filaments and promoting their depolymerization at both ends. Current Biology 27, 1956-1967.July 10, 2017.
Hung, C., Lee, C., Chang, C., Jong, Y., Chen, C., Hseih, W., Su, Y. and Lin, W. (2008) Genotyping of the G1138A mutation of the FGFR3 gene in patients with achondroplasia using high-resolution melting analysis. Clin. Biochem. 41: 162-166.
Kennerson, M., Warburton, T., Nelis, E., Brewer, M., Polly, P., De Jonghe, P., Timmerman, V. and Nicholson, G. (2007) Mutation scanning the GJB1 gene with high-resolution melting analysis: implications for mutation scanning of genes for Charcot-Marie-Tooth disease. Clin. Chem. 53, 349-352.
Li, S., Liu, S., Liu, Y., Lu, H., Tan, Y., Huang, J., Wei, P., and Shu, Q.Y. (2018). HRM-facilitated rapid identification and genotyping of mutations induced by CRISPR/Cas9 mutagenesis in rice. Crop Breed. Appl. Biotechnol. 18(2): 184-191.
Martin-Ortigosa, S. and Wang, K. (2014). Proteolistics: a biolistic method for intracellular delivery of proteins. Transgenic Res. 23(5):743-756.
McKinney, J., Longo, N., Hahn, S., Matern, D., Rinaldo, P., Strauss, A. and Dobrowolski, S. (2004) Rapid, comprehensive screening of the human medium chain acyl-CoA dehydrogenase gene. Mol. Genet. Metab. 82, 112-120.
Nan Q, Dong Qian, Yue Niu, Yongxing He, Shaofei Tong, Zhimin Niu, Jianchao Ma, Yang Yang, Lizhe An, Dongshi Wan, Yun Xiang (2017) Plant Actin-Depolymerizing Factors possess opposing biochemical properties arising from key amino acid changes throughout evolution. The Plant Cell, 29: 395-408.
Padilla-Martinez JP, Berrospe-Rodriguez C, Aguilar G, Ramirez-San-Juan JC and Ramos-Garcia R (2014). Optic cavitation with CW lasers: A review. Physics of Fluids 26, 122007.
Pospíšilová, J., Wilhelmová, N., Synková, H., Čatský, J., Krebs, D., Tichá, I., Hanáčková, B., Snopek, J. (1998). Acclimation of tobacco plantlets to ex vitro conditions as affected by application of abscisic acid. - J. exp. Bot. 49: 863-869.
Sack M, Rademacher T, Spiegel H, Boes A, Hellwig S, Drossard J, Stoger E and Fischer R (2015). From gene to harvest: insights into upstream process development for the GMP production of a monoclonal antibody in transgenic tobacco plants. Plant Biotechnology Journal 13:1094-105.
Willmore, C., Holden, J., Zhou, L., Tripp, S., Wittwer, C. and Layfield, L. (2004) Detection of c-kit-activating mutations in gastrointestinal stromal tumors by high-resolution amplicon melting analysis. Am. J. Clin. Pathol. 122, 206-216.
Zhou, L., Vandersteen, J., Wang, L., Fuller, T., Taylor, M., Palais., B. and Wittwer, C. (2004) High-resolution DNA melting curve analysis to establish HLA genotypic identity. Tissue Antigens 64, 156-164.

## Claims

1. A method for altering the genome of an intact plant cell without inserting exogenous genetic material into the genome comprising:
(i) providing an intact plant cell that comprises an endogenous gene to be modified;
(ii) providing a genome-modifying formulation comprising a pre-assembled ribonucleoprotein (RNP)-complex comprising a nucleic acid-modifying protein and ribonucleic acid, wherein the ribonucleic acid is labeled with a visual marker;
(iii) delivering said RNP-complex into the plant cell by a laser-assisted transfection method, wherein the cell is surrounded by a liquid and the laser focal point is in the liquid adjacent to the cell wall and not directly in contact with the cell wall;
(iv) inducing one or more single or double stranded DNA breaks in the cell genome to produce a cell having a detectable targeted genomic modification without the presence of any exogenous genetic material in the cell genome.

2. The method according to claim 1, wherein the method comprise the further step of:
(v) identifying the cells transfected with the RNP-complex by microscopy, in particular by fluorescence microscopy.

3. The method according to anyone of claims 1 to 2, wherein the method comprises the further step of:
(vi) selecting the cells transfected with the RNP-complex and optionally regenerating the cells to intact plantlets by growing in and/or on a growth media.

4. A method to produce genome edited plants without inserting exogenous genetic material into the genome of the plant comprising the steps of:
(i) providing a plant explant isolated by any organ and tissue of a plant and comprising intact plant cells comprising an endogenous gene to be modified;
(ii) providing a genome-modifying formulation comprising a pre-assembled ribonucleoprotein (RNP)-complex comprising a nucleic acid-modifying protein and ribonucleic acid, wherein the ribonucleic acid is labeled with a visual marker;
(iii) layering the plant explant onto the RNP-complex formulation in a transparent support;
(iv) delivering said RNP-complex into a plant cell of the plant explant by laser-assisted transfection, wherein the cell is surrounded by a liquid and the laser focal point is in the liquid adjacent to the cell wall and not directly in contact with the cell wall;
(v) inducing one or more single or double stranded DNA breaks in the cell genome to produce a cell having a detectable targeted genomic modification without the presence of any exogenous genetic material in the cell genome.
(vi) identifying the cells transfected with the RNP-complex by microscopy, in particular by fluorescence microscopy;
(vii) selecting the cells transfected with the RNP-complex from the plant explant
(viii) regenerating the cells to intact plantlets by growing in and/or on a growth media.

5. The method according to claim 4, wherein the method comprises the further step of:
(ix) analyzing the plantlets to confirm editing of the target gene using any method for genetic characterization of genome-edited plants such as high-resolution melt analysis for identifications of mutated alleles;
(x) selecting the analyzed plantlets scoring positive and further growing those plantlets to full plants.

6. The method according to any one of claims 1 to 5, wherein the laser irradiation conditions are first adjusted before delivering of the RNP-complex by the laser-assisted transfection method.

7. The method according to any one of claims 1 to 6, wherein the plant cell comprises a cell wall, in particular said plant cell is from a plant from the *Solanaceae* family, like *Nicotiana tabacum* and *Nicotiana benthamiana* from the genus *Nicotiana.*

8. The method according to any one of claims 1 to 7, wherein the nucleic acid modifying protein is a CRISPR-Cas9 endonuclease, a CRISPR-Cpf1 nuclease or a CRISPR-C2c2 endoribonuclease or any other designer nuclease.

9. The method according to any one of claims 1 to 8, wherein the ribonucleic acid comprises a crRNA and a tracrRNA, or a chimeric cr/tracrRNA hybrid.

10. The method according to any one of claims 1 to 9, wherein the RNP-complex comprises a crRNA, a tracrRNA and a CRISPR-Cas9 endonuclease that target to a DNA sequence that is endogenous to the cell and that induces a double strand break at or near the sequence to which the crRNA and tracrRNA sequence is targeted to, or at or near the sequence to which the cr/tracrRNA is targeted to.

11. The method according to any one of claims 1 to 10, wherein the cell is exposed to the pre-assembled ribonucleoprotein (RNP)-complex, wherein after delivering said RNP-complex into the cell, the genome of the cell is altered/modified, and wherein after the modification/alteration the cells are further cultivated to derive a cell, cell line, tissue or organism with a modified (altered) genome without the presence of a marker gene.

12. The method according to any one of claims 1 to 11 wherein the laser is a multiphoton laser operating under pulsing conditions and wherein the laser power is between 0.5 W and 3 W, preferably about 2 W, more preferably wherein the laser power is between 65 and 90%, preferably at 70% of 2 W and wherein preferably the wavelength is between 700 nm und 900 nm, preferably 800 nm.

13. The method according to any one of claims 1 to 12, wherein the genome-modifying formulation comprises a plurality of different pre-assembled ribonucleoprotein (RNP)-complexes, in particular wherein the ribonucleoprotein (RNP)-complexes differ in the nucleic acid sequence of the ribonucleic acid comprised in the complexes.

14. The method according to any one of claims 1 to 13, wherein the genome-modifying formulation comprises at least two different pre-assembled ribonucleoprotein (RNP)-complexes, in particular wherein the two different pre-assembled ribonucleoprotein (RNP)-complexes target different target sequences in the genome.


SEQUENCE LISTING

<110>  Fraunhofer-Gesellschaft zur Förderung der angewandten
       Forschung e.V.

<120>  METHODS FOR THE PRODUCTION OF GENOME EDITED PLANTS

<130>  FRA-PA38-EP

<160>  14

<170>  PatentIn version 3.5

<210>  1
<211>  23
<212>  DNA
<213>  Nicotiana tabacum

<400>  1
ttttttgga atatcaggtt tgg                                              23

<210>  2
<211>  23
<212>  DNA
<213>  Nicotiana tabacum

<400>  2
cttggagctg aagaggaaga agg                                             23

<210>  3
<211>  21
<212>  DNA
<213>  Artificial Sequence

<220>
<223>  adf gene primer sequence 1

<400>  3
atgtctttca gattcagagg g                                               21

<210>  4
<211>  18
<212>  DNA
<213>  Artificial Sequence

<220>
<223>  adf gene primer sequence 2

<400>  4
tcagtgagcg cggtcttt                                                   18

<210>  5
<211>  21
<212>  DNA
<213>  Artificial Sequence

<220>
<223>  pds gene primer sequence 1

<400>  5
cttgattttg tgggtgaagg a                                                21


<210>  6
<211>  20
<212>  DNA
<213>  Artificial Sequence

<220>
<223>  pds gene primer sequence 2

<400>  6
gcaaggcaga atacagatcg                                                  20


<210>  7
<211>  20
<212>  DNA
<213>  Artificial Sequence

<220>
<223>  pds HRM

<400>  7
atctggctga tgctggtcac                                                  20


<210>  8
<211>  26
<212>  DNA
<213>  Artificial Sequence

<220>
<223>  pds HRM 2 primer

<400>  8
aaggaataaa attaaaggaa agcatg                                           26


<210>  9
<211>  20
<212>  DNA
<213>  Artificial Sequence

<220>
<223>  pds SEQ primer sequence 1

<400>  9
ccattgaccg gttagcagtt                                                  20


<210>  10
<211>  24
<212>  DNA
<213>  Artificial Sequence

<220>
<223>  pds SEQ primer sequence 1

<400>  10
tgaacaccct tgcaattgtt tgag                                             24

<210>  11
<211>  20
<212>  DNA
<213>  Artificial Sequence

<220>
<223>  adf HRM primer sequence 1

<400>  11
ttctggcatg ggtgtagctg                                                    20


<210>  12
<211>  20
<212>  DNA
<213>  Artificial Sequence

<220>
<223>  adf HRM primer sequence 1

<400>  12
gctgccagtt ttctcaacaa                                                    20


<210>  13
<211>  21
<212>  DNA
<213>  Artificial Sequence

<220>
<223>  adf SEQ primer sequence 1

<400>  13
agtcaaggtg cctgcaattt a                                                  21


<210>  14
<211>  23
<212>  DNA
<213>  Artificial Sequence

<220>
<223>  adf SEQ primer sequence 2

<400>  14
caaagaaaat cttgctcttt tgg                                                23